# EUROPEAN PATENT APPLICATION

(11) **EP 4 321 205 A1**
(43) Date of publication of application: **14.02.2024**
(21) Application number: 22190083.0
(22) Date of filing: 12.08.2022
(51) Int. Cl.: A61N 1/05, A61L 2/26

(54) **PROTECTIVE CASING FOR A THIN-FILM LEAD PADDLE**

(71) Applicant: INBRAIN Neuroelectronics SL, 08028 Barcelona (ES)
(72) Inventor: BAKKER, Bert, 4261 LH Wijk en Aalburg (NL); Vico Martino, Oscar, 08290 Cerdanyola del Vallès (ES)
(74) Representative: DTS Patent- und Rechtsanwälte Schnekenbühl und Partner mbB

(57) **Abstract**

The present invention relates to a protective casing (10) for a thin-film lead paddle (102) of a surgical lead (100), the thin-film lead paddle (102) being connected to a lead cable (104) through an interconnection portion (106), the protective casing (10) comprising:
a housing (12) formed by a first part (20) and at least a second part (30), the housing (12) being configured and adapted to enclose at least a part of the thin-film lead paddle (102) of the surgical lead (100),
wherein each of the first and second parts (20, 30) includes a first surface (22, 32) and a second surface (24, 34), the second surfaces (24, 34) of the first and second detachable parts (20, 30) being at least partially in contact to one another and/or in contact with the thin-film lead paddle (102) when the first and second detachable parts (20, 30) are held in place to form the housing (12).

Furthermore, the present invention relates to a system with such a protective casing (10) and a method for sterilizing a surgical lead (100) comprising a thin-film lead paddle (102) by using such a protective casing (10).

## Description

The present invention belongs to the technical field of surgical leads.

Surgical leads are known in the art.

Usually, surgical leads include a lead paddle and a lead cable, which can be made from the same materials or different materials.

The lead paddle and the lead cable may be connected to one another, for example through an interconnection portion.

Alternatively, the lead paddle and the lead cable can be made in one piece.

An electrode area is formed at the distal end of the lead paddle for delivery of electrical stimulation to, or recording/sensing of neural potentials of a target site within a patient's body.

Surgical leads are invasive. For instance, surgical leads may be adapted to be implanted at one or more target sites of the patient's body by surgery.

Lead paddles can be made of a thin film of flexible material, making it highly flexible.

Here, for "thin film" it is intended a film having a thickness that is less than 200 µm, preferably less than 100 µm

Thin-film lead paddles are highly sensitive to mechanical stress, and are thus exposed to damages during transport, cleaning, and sterilization operations, or when otherwise handled by an operator.

There are several necessary steps that need to be implemented after manufacturing before a surgical lead is ready for its use in the operation room.

In particular, surgical leads may need to be tested, cleaned, sterilized and packaged. After packaging, the surgical leads are transported to warehouses, hospitals or the like and thereby stored. Then, the leads are brought to the operation room, where they are unpackaged and made ready for use.

Each of the above specified steps may expose the thin-film lead paddle of the surgical lead to mechanical stress which could lead to damages.

Hence, there is a need for a solution allowing to effectively reduce the risk of damages to the thin-film lead paddle of a surgical lead during handling, testing, cleaning, sterilization, transport, packaging/unpackaging and any other step that needs to be implemented before use in the operation room.

It is therefore an object of the present invention to provide a protective casing for a thin-film lead paddle allowing to protect the thin-film lead paddle against damages during handling, testing, cleaning, sterilization, transport, packaging/unpackaging and any other step that is necessary prior to use in the operation room.

This object is solved with a protective casing with the features of claim 1. Accordingly, a protective casing for a thin-film lead paddle of a surgical lead is provided, wherein the thin-film lead paddle is connected to a lead cable through an interconnection portion, the protective casing comprising:
a housing comprising or including a first part and at least a second part, the housing being configured and adapted to enclose at least a part of the thin-film lead paddle of the surgical lead,
wherein each of the first and second parts includes a first surface and a second surface, the second surfaces of the first and second parts being at least partially in contact to one another and/or in contact with the thin-film lead paddle during use.

The present invention provides a protective casing for a thin-film lead paddle of a surgical lead.

The thin-film lead paddle can be connected to a lead cable through an interconnection portion.

Alternatively, the thin-film lead paddle and the lead cable can be made in one piece.

The protective casing includes a housing.

The housing can be configured and adapted to enclose the thin-film lead paddle and the interconnection portion of the surgical lead.

The housing includes a first part and at least a second part.

Advantageously, the housing may include two parts, namely the first part and the second part described above.

Alternatively, the housing may include three or more parts.

The first and second parts include a first surface.

The first and second parts further include a second surface.

The second surface of the first part and the second surface of the second part are in contact to one another during use.

Accordingly, the thin-film lead paddle and the interconnection portion are stably retained between the second surface of the first part and the second surface of the second part.

Each of the first and second parts have a proximal end and a distal end.

The proximal end of the first part and the proximal end of the second part are configured for connection to the lead cable.

In particular, the proximal end of the first part and the proximal end of the second part can be adapted to enclose a distal portion of the lead cable, proximal to the interconnection portion.

Accordingly, the protective casing secures the surgical lead.

Advantageously, at least one of the first part or the second part forming the housing is permeable to fluids and/or gases, especially when the thin-film lead paddle of a surgical lead is placed in the protective casing and being sterilized and/or tested in the protective casing.

Surgical leads are electrically tested while being immersed in a medium such as a saline solution.

When for example the thin-film lead paddle is immersed into the saline solution for testing, the saline solution is allowed to penetrate through the permeable first part and/or second part forming the housing, and thereby contact the thin-film lead paddle so that testing can be performed.

Once testing is completed, the thin-film lead paddle and the attached protective casing are removed from the saline solution, and a cleaning step is performed to remove residual saline solution from the thin-film lead paddle and the protective casing.

Since testing and subsequent cleaning operations can be carried out while the protective casing is mounted to the surgical lead, the risk of damages to the thin-film lead paddle during testing and/or cleaning can be significantly reduced.

The invention is based on the basic idea that, by providing a protective casing for a thin-film lead paddle of a surgical lead, it is possible to provide a protection for the thin-film lead paddle during handling, testing, cleaning, sterilization, transport, packaging/unpackaging and any other step that need to be implemented prior to use in the operation room. The protective casing allows to protect the thin-film lead paddle from bending, kinking and/or against mechanical stress.

Additionally, permeability of the first part and/or the second part forming the housing allows fluids and/or gases (e.g., cleaning fluids, sterilization fluids, activation fluids, saline, or the like) to penetrate through the housing and thereby contact the thin-film lead paddle. Accordingly, cleaning and/or sterilization and/or testing operations can be carried out while the protective casing is mounted to the surgical lead, so that the risk of damages to the thin-film lead paddle can be largely prevented.

The first and second parts forming the housing may be detachable.

Alternatively, the first and second parts forming the housing may be connected to one another through a connection means.

Preferably, said connection means includes a hinge.housing is made in one piece. Furthermore, it is possible that the protective casing comprises at least one locking element for holding the first and second parts in place with respect to one another to form the housing.

The locking element may be configured to break when separated from the housing.

In this case, the protective casing cannot be reused.

Alternatively, the locking element may be configured to be removed from/connected to the housing multiple times.

This renders the protective casing suitable for multiple re-use.

With the at least one locking element it is possible to secure the parts of the protective casing in the correct position and to provide a stable and easy to use protective casing.

It is possible that the at least one locking element is formed integral with the first part and/or the second part.

This allows to prevent the at least one locking element from accidentally falling or otherwise get lost.

Further, this also renders the protective casing easier to handle.

It is possible that the at least one locking element comprises or consists of at least one clip element, especially wherein the clip element is configured to hold the first and second parts in place by clipping.

The clip element may comprise (at least partially) a U-shaped cross-section.

Clipping of the locking elements is an easy and safe way and will facilitate the handling of the locking elements even in sterile environments, where for example the users must wear gloves or other protective clothing.

Accordingly, the protective casing can be easily assembled and removed by the operator without the risk of causing stress or damages to the thin-film lead paddle.

The locking element may also be or comprise a sleeve-like element. Such a sleeve-like element may be slid at least partially over the protective casing.

Further, it is possible that there is a spring-lock element. Here, a locking element can be actuated by a spring, thereby allowing an easy opening of the protective casing. Similar mechanisms are e.g. known from eye-wear (or sunglasses) casings.

It is possible, that the first surface and/or the second surface of the first and second parts includes a hole pattern structure, especially a bee-cell-hole pattern structure.

Via the holes, the fluids and/or gasses needed for sterilization and cleaning may be routed to the thin film of the surgical lead, while still a huge stability and mechanical protection can be provided.

Preferably, the bee-cell-hole pattern structure is provided on both the first and second surfaces of the first and second parts.

The bee-cell-hole pattern structure allows to facilitate cleaning and/or sterilization operations.

Further, the presence of the bee-cell-hole pattern structure allows to keep force protection.

Preferably, the bee-cell-hole pattern structure is formed over the entire first surface and/or second surface of the first and second parts.

The hole pattern structure is formed over almost the entire first surface and/or almost the entire second surface of the first and/or second parts.

More preferably, the bee-cell-hole pattern structure is formed over the entire first surface and the entire second surface of the first and second parts.

Advantageously, the first part and the second part forming the housing are made of biocompatible polymer material, permeable to fluids and gases. In particular, the polymer material may include or consist of a biocompatible polymer, especially a medical grade polymer.

Such polymer can be inter alia but not limited to Polyamide (PA), especially PA 2200, Polypropylene (PP), Poly ethylene (PE), Poly ethylene terephthalate (PET), Poly-ether-ether-ketone (PEEK), Polyphenylsulfon (PPSU), Polyoxymethylene (POM), Polyethylenimin (PEI) and the like.

The polymer material can be e.g. processed by means of additive manufacturing. Also, injection molding can be for example used as a manufacturing process for this polymer.

Advantageously, the housing may have a rectangular shape.

Preferably, such a rectangularly-shaped housing has substantially round edges.

This allows to prevent damages to a sterile packaging when the surgical lead and the protective casing are packaged for transport and storage purposes.

Further, the presence of substantially round edges allows to prevent possible damages to gloves worn by an operator, such as a doctor or a nurse, while manipulating the protective casing for use. The first and second parts are provided with a respective recessed portion.

In particular, the recessed portions are formed on the second surface of the first and second parts, in the proximity of the proximal end.

Accordingly, when the first and second parts are connected to one another to form the housing, the recessed portions of the first and second parts define a cavity for housing the interconnection portion of the surgical lead.

With this, the lead can be placed correctly and very easily only in one possible way in the protective cases. This increases the safety of the handling of the casing and therefore of the whole system.

The protective casing can be disposable.

Alternatively, the protective casing can be adapted for re-use.

In such a case, the protective casing is sterilized and then returned to the manufacturer or a laboratory for re-use.

Furthermore, it is possible that one part among the first and second parts comprises a form-fit protrusion (e.g. a projection) and the other part among the first and second parts comprises a form-fit recess (e.g. a groove), which are configured to engage with each other for a form-fit. This way, the position of the parts of the protective casing can be facilitated and the handling is further simplified.

The distal end of the first part may include a projection.

Correspondingly, the distal end of the second part may include a groove.

In particular, the projection is configured to engage with the groove when the first and second detachable parts are held in place to form the housing.

The projection and corresponding groove allow to facilitate correct positioning of the first and second parts to form the housing.

In addition, through the engagement of the projection and the groove, the first and second parts are more reliably maintained in place during use.

Additionally, the protective casing can comprise an adjustment zone for holding the lead cable, wherein the adjustment zone is at least partially formed by the first and second parts, especially wherein the first part and/or the second part comprises an adjustment zone recess. In the adjustment zone recess the lead cable can be safely and easily fixated and positioned.

The adjustment zone recess further has an advantageous strain relieve function.

Advantageously, the protective casing may further include an optical inspection window.

Preferably, said optical inspection window can be made of a transparent polymer material.

Even more preferably, the transparent polymer material is biocompatible.

This ensures compatibility with living tissues that may come into contact with the optical inspection window during use.

Advantageously, a barcode or a radio-frequency identification (RFID) code can provided on the protective casing.

This allows for easy and reliable traceability of the protective casing.

Advantageously, an identification number and/or indications for use and/or warnings, can be provided on the protective casing.

This allows for a more intuitive use of the protective casing.

The present invention further provides a system including the protective casing as described above and a surgical lead.

The surgical lead comprises a thin-film lead paddle and a lead cable, for example connected though an interconnecting portion.

Alternatively, the thin-film lead paddle and the lead cable can be in one piece.

Advantageously, the system may further include a protection means made of a soft material or a soft filler.

The protection means is provided at the connection point between the housing and the lead cable.

Accordingly, mechanical stress at the connection point can be significantly reduced.

Also, the present invention relates to a method for handling a surgical lead comprising a thin-film lead paddle.

The method can comprise at least the following steps:
- covering the thin-film lead paddle with a protective casing, especially wherein the protective casing is a protective casing as described above;
- handling at least the thin-film lead paddle while being in the protective casing.

Also, the present invention relates to a method for sterilizing a surgical lead comprising a thin film lead paddle.

The method can comprise at least the following steps:
- covering the thin-film lead paddle with a protective casing, especially wherein the protective casing is a protective casing as described above and wherein at least one of the first part or the second part forming the housing is permeable to fluids and/or gases;
- performing a sterilization operation of the thin-film lead paddle while being in the protective casing by allowing a sterilizing fluid or a sterilizing gas to penetrate inside the casing through the permeable first part and/or second part.

Further details and advantages of the present invention shall now be disclosed in connection with the drawings.

It is shown in
- **Fig. 1**: a perspective view showing a protective casing according to an embodiment of the invention, including a housing formed of first and second detachable parts, and a locking means for connecting the first and second detachable parts to one another;
- **Fig. 2**: a perspective view showing a surgical lead including a thin-film lead paddle, a lead cable and an interconnection portion, where a protection means is provided at the distal end of the lead cable, proximal to the interconnection portion;
- **Fig. 3**: a perspective view of the protective casing of **Fig.** 1, showing only the second detachable part with the surgical lead 100 placed in it; and
- **Fig. 4**: a side view in partial cross section of a system including the surgical lead of **Fig.** 2 and the protective casing of **Fig. 1****.**

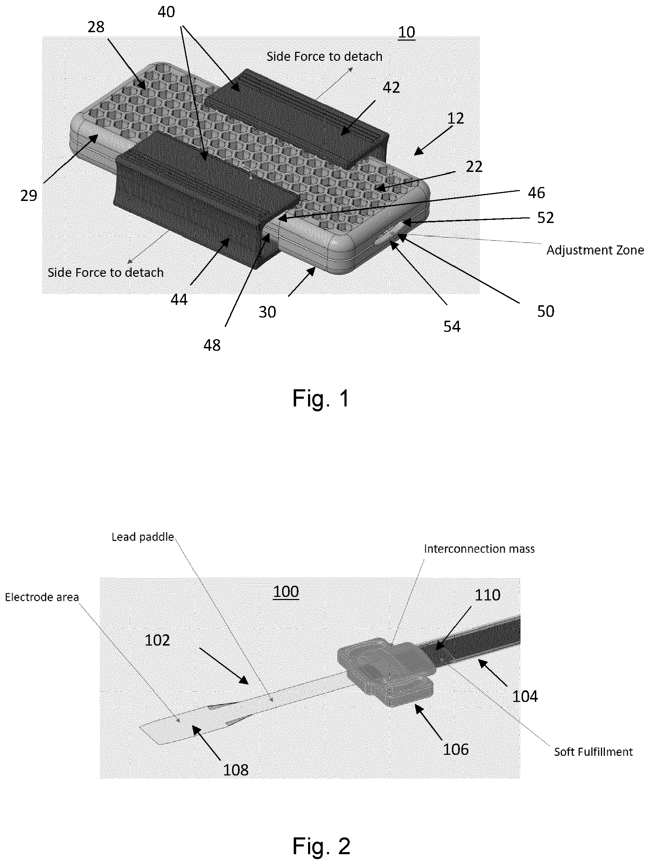

**Fig.** 1 shows a possible embodiment of a protective casing 10 for a thin-film lead paddle of a surgical lead, such as the surgical lead 100.

The protective casing 10 includes a housing 12.

In the shown embodiment, the housing 12 has a first detachable part 20.

The first detachable part 20 has a first surface 22.

The first surface 22 is forming the outside of the first detachable part 20.

Further, there is a second surface 24 of the first detachable part 20, which is forming the outside of the first detachable part 20.

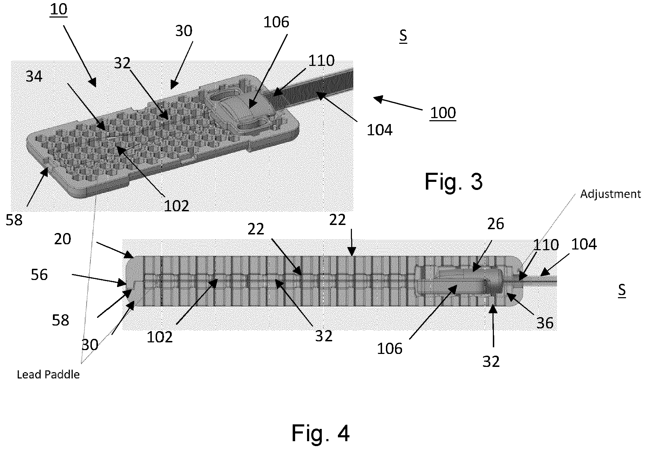

The second surface 24 is at least partially adapted to the form of the lead paddle 102 (cf. Fig. 2 and Fig. 3).

This adaption of the second surface 24 is provided by a recessed portion 26 of the first detachable part 20.

In the shown embodiment, the first detachable part 20 is permeable to fluids and/or gases.

In the shown embodiment, the first detachable part 20 has a bee-cell-hole pattern structure 28.

Also, the first detachable part 20 has a frame structure 29.

The frame structure 29 encircles the bee-cell-hole pattern structure 28.

Further the housing 12 has a second detachable part 30.

In the shown embodiment, the second detachable part 30 is also permeable to fluids and/or gases.

Also, the second detachable part 30 has a first surface 32 (cf. also Fig. 4).

The first surface 32 is forming the outside of the first detachable part 30.

Further, there is a second surface 34 of the first detachable part 30, which is forming the outside of the first detachable part 30.

The second surface 34 is also at least partially adapted to the form of the lead paddle 102 (cf. **Fig. 2** and **Fig. 3****).**

This adaption of the second surface 34 is provided by a recessed portion 36 of the second detachable part 30.

Moreover, the second detachable part 30 has also a bee-cell-hole pattern structure 38.

Further, the second detachable part 30 has a frame structure 39.

The frame structure 39 encircles the bee-cell-hole pattern structure 38.

The bee-cell-hole pattern structures 28, 38 have open end-to-end holes, connecting the first surface 22, 32 with the second surface 24, 34, which allow fluid and gas to enter the protective casing 10 from the outside and also to leave the protective casing 10.

In the shown embodiment, the housing 12 is formed by two parts, namely the first detachable part 20 and the second detachable part 30 described above.

Not shown is that the housing 12 may as well be formed by three or more parts.

For instance, said three or more parts may be detachable.

Alternatively, said three or more parts may be non-detachable.

The protective casing 10 is further equipped with locking elements 40.

In the shown embodiment, the locking elements 40 are formed as clips and there is in this example embodiment a first clip element 42 and a second clip element 44.

Not shown is that the at least one locking element 40 may as well be formed as an integral part of the first detachable part 20 and/or the second detachable part 30.

For the locking elements 40 there are respective recesses 46, 48 in the outer part of the frame structures 29, 39. The locking elements 40 fit into the recesses 46, 48.

The protective casing 10 has an adjustment zone 50.

The adjustment zone 50 has an oval form.

Further, the adjustment zone 50 forms a through hole from the outside of the protective casing 10 to the inside of the through hole.

The adjustment zone 50 is formed by adjustment zone recesses 52, 54 in the frame structures 29, 39 of the first detachable part 20 and second detachable part 30.

The first detachable part 20 and the second detachable part 30 fit to each other.

In particular, the dimensions and the form of the first detachable part 20 and the second detachable part 30 are more or less the same and they are configured such that they can be placed upon each other.

Further, there is a form-fit protrusion 56 in the inner part of the frame structure 29 of the first detachable part 20 and there is a form-fit recess 58 in the inner part of the frame structure 39 of the second detachable part 30.

The form-fit protrusion 56 fits into the form-fit recess 58. Especially, they can form-fit into each other.

The form-fit protrusion 56 and the form-fit recess 58 are configured such that by fitting into each other the first detachable part 20 and the second detachable part 30 are secured at least partially into their position relatively to each other, such that the first detachable part 20 and the second detachable part 30 can form a secure enclosure for the surgical lead, especially for a thin-film lead paddle of a surgical lead 100.

The material of the protective casing 10 is a medical grade polymer.

In particular, the polymer material may include or consist of a medical grade polymer. Such polymer can be inter alia but not limited to Polyamide (PA), especially PA 2200, Polypropylene (PP), Poly ethylene (PE), Poly ethylene terephthalate (PET), Poly-ether-ether-ketone (PEEK), Polyphenylsulfon (PPSU), Polyoxymethylene (POM), Polyethylenimin (PEI) and the like.

The polymer material can be e.g. processed by means of additive manufacturing. Also, injection molding can be for example used as a manufacturing process for this polymer.

In the shown embodiment, all parts of the protective casing 10 (i.e. the first and second detachable parts 20, 30 and also the first clip element 42 and second clip element 44) are made of PA 2200 and the manufacturing is done by means of additive manufacturing (3D printing) or injection molding.

As shown in Fig. 2, the surgical lead 100 has a thin-film lead paddle 102.

Furthermore, the surgical lead 100 has a lead cable 104.

The lead cable 104 is connected with the thin-film lead paddle 102 with an interconnection portion 106.

The thin-film lead paddle 102 has an electrode area 108 of the thin-film lead paddle.

The distal end of the lead cable 104 is adjacent to the interconnection portion 106 equipped with a protection element 110. The protection element 110 fits into the adjustment zone 50 of the protective casing 10.

In the shown embodiment, the protection element 110 is a ring made of a soft material or a soft filler, which surrounds the distal end of the lead cable 104, proximal to the interconnection portion 106 **(****Figs. 1****,** **3** and **4****).**

Further, the interconnection portion 106 is adapted to the recessed portion 26 of the first detachable part 20 and further adapted recessed portion 36 of the second detachable part 30.

The lead paddle 102 is made of a highly-flexible thin film, having a thickness in the micrometric range.

Accordingly, the thin-film lead paddle 102 is particularly sensitive to mechanical stress.

An electrode area 108 is formed at the distal end of the thin-film lead paddle 102 for delivery of electrical stimulation to a target site within the body of a patient.

The functionality of the protective casing 10 is as follows:
The protective casing 10 provides protection for the thin-film lead paddle 102 during handling, testing, cleaning, sterilization, transport, packaging/unpackaging and any other step that needs to be implemented prior to use of the surgical lead 100 in the operation room.

The housing 12 is configured and adapted to enclose the thin-film lead paddle 102 and the interconnection portion 106 of the surgical lead 100 (cf. Fig. 3 and Fig. 4).

The locking elements 40 in form of the clips 42, 44 are configured to connect the first and second detachable parts 20, 30 to form the housing 12, and maintain the first and second detachable parts 20, 30 in place with respect to one another.

In the present embodiment, the locking means 40 includes a first clip element 42 and a second clip element 44, the first and second clip elements 42, 44 being configured to connect the first and second detachable parts 20, 30 to one another by clipping (cf. Fig. 1).

Hence, the protective casing 10 can be easily and safely assembled and removed by the operator without causing any stress or damages to the lead portion 102.

Each of the first and second detachable parts 20, 30 includes a first surface 22, 32 and a second surface 24, 34. In particular, when the first and second detachable parts 20, 30 are connected to form the protective casing 10, the second surfaces 24, 34 of the first and second detachable parts 20, 30 are brought into contact to one another (Figs. 2-3).

Accordingly, the thin-film lead paddle 102 can be stably retained in place within the protective casing 10 and can be prevented from bending or kinking.

The proximal end of the first detachable part 20 and the proximal end of the second detachable part 30 are configured for connection to the lead cable 104.

In particular, the proximal ends of the first and second detachable parts 20, 30 are configured to enclose the distal portion of the lead cable 104, proximal to the interconnection portion 106, thereby securing the protective casing 10 to the surgical lead 100 (cf. **Fig. 3** and **Fig. 4****).**

The permeability of the protective casing 10 allows to clean and/or sterilize the thin-film lead paddle 102 while the protective casing 10 is mounted to the surgical lead 100.

In particular, a cleaning and/or sterilizing fluid is allowed to pass through the permeable holes of the especially a bee-cell-hole pattern structure 28, 38, and thereby approach and contact the thin-film lead paddle 102.

Due to the presence of the protective casing 10, the risk of damages to the thin-film lead paddle 102 during cleaning and/or sterilizing operations can be largely avoided.

The permeability of the housing 12 is also advantageous as it allows to perform testing while the protective casing 10 is mounted to the surgical lead 100.

In particular, when the thin-film lead paddle 102 is immersed into medium such as a saline solution for testing, the saline solution is allowed to penetrate through the permeable bee-cell-hole pattern structure 28, 38, and thereby contact the thin-film lead paddle 102, so that testing can be performed.

Once testing is completed, the thin-film lead paddle 102 and the attached protective casing 10 are removed from the saline solution, and a cleaning step is performed to remove residual saline solution from the thin-film lead paddle 102 and the protective casing 10.

Since testing and subsequent cleaning operations can be carried out while the protective casing 10 is still mounted to the surgical lead 100, the risk of damages to the thin-film lead paddle 102 during testing and/or cleaning can be significantly reduced.

In the shown embodiment, both the first surface 22, 32 and the second surface 24, 34 of the first and second detachable parts 20, 30 is provided with a bee-cell-hole pattern 30 **(****Figs. 2** to **4****).**

The bee-cell-hole pattern structure 28, 38 allows facilitating cleaning and/or sterilization operations.

Further, the bee-cell-hole pattern structure 28, 38 allows to keep force protection.

In the present embodiment, the protective casing 10 has a rectangular shape **(****Figs.** 2 and **4****).**

Not shown is that the protective casing 10 may also have a different shape (e.g., square, oval or circular), as far as it is suitable for the purpose.

In the present embodiment, the protective casing 10 has substantially round edges.

This has the advantage that possible damages to a sterile packaging enclosing the protective casing 10 and the surgical lead 100 can be largely prevented.

In the present embodiment, the first and second detachable parts 20, 30 are provided with a respective recessed portion 26, 36, formed on the second surface 24, 34 in the proximity of the proximal end (Figs. 3-4).

When the first and second detachable parts 20, 30 are connected to one another to form the protective casing 10, the recessed portion 26 of the first detachable part 20 and the recessed portion 36 of the second detachable part 30 define a cavity the interconnection portion 106, as shown in Fig. 3 and Fig. 4.

Accordingly, the thin-film lead paddle 102 and the interconnection portion 106 can be stably retained in place within the housing 12.

The protective casing 10 may be disposable.

Alternatively, the protective casing 10 can be adapted for re-use. In such a case, the protective casing 10 is sterilized and then returned to the manufacturer or a laboratory for re-use or inspection.

In the present embodiment the distal end of the first detachable part 20 includes a projection 56 or a form-fit protrusion 56 (Fig. 4), while the distal end of the second detachable part 30 includes a groove 58 or form-fit recess 58 (Figs. 3-4).

As shown in Fig. 4, when the first and second detachable parts 20, 30 are connected to form the protective casing 10, the projection 56 engages with the groove 58.

The projection 56 and corresponding groove 58 allow to facilitate correct positioning of the first and second detachable parts 20, 30 to form the protective casing 10 by correctly positioning the first and second detachable parts 20, 30 relatively to each other.

In addition, through the engagement of the projection 56 and the groove 58, the first and second detachable parts 20, 30 are more reliably maintained in place after connection.

Not shown is that the protective casing 10 may be provided with an optical inspection window.

Preferably, said optical inspection window is made of a transparent polymer material.

Even more preferably, said optical inspection window is made of a transparent polymer material which is biocompatible.

Not shown is also that a barcode or a radio-frequency identification (RFID) code can provided on the protective casing 10 for traceability purposes.

Fig. 4 shows a system S including the surgical lead 100 and the protective casing 10 described above.

The protective casing 10 is connected at its proximal end to a distal end of the lead cable 104.

Advantageously, the system S may further include a protection element 110 such as a soft material or a soft filler, provided at the connection point between the housing 12 and the lead cable 200.2 of the surgical lead 200 **(****Figs. 1****,** **3** and **4****).**

The protective casing 10 provides protection for the thin-film lead paddle 102 during handling, testing, cleaning, sterilization, transport, packaging/unpackaging and any other step that needs to be implemented before use in the operation room.

This way, the protective casing 10 is easy to assemble and to remove, so no stress by the casing 10 is caused and a surgeon or a nurse with (double) gloves is able to remove the casing 10 without causing stress and damages. The casing 10 locking mechanism with the locking elements 40 may not have small parts which can get lost or with force release jump away from the device.

The thin film of the surgical lead 100 will touch the casing 10, and this may not damage the by touching this casing 10.

The casing 10 is of biocompatible, sterilizable and chemical resistant material as specified above and manufactured such no biohazards are/will be created.

Next to the mechanical properties, the casing 10 is due to its structure permeable for sterilization gasses and fluids, such the casing 10 and the lead 100 get properly sterilized. To electrically test the lead 100, the lead-paddle 102 can be submerged into saline. If the casing 10 is already attached, the casing 10 does allow the saline to touch the electrodes of the lead 100 such the test can be executed. Afterwards a cleaning step to remove the saline is required.

The casing 10 can be packaged with the lead into one package. During the transport and packaging steps, the lead-paddle 102 is protected. After unboxing and unwrapping, the protective casing 10 is removed before use.

### References

- 10: Protective casing
- 12: Housing

- 20: First (detachable) part
- 22: First surface of the first (detachable) part
- 24: Second surface of the (first detachable) part
- 26: Recessed portion of the first (detachable) part
- 28: (Bee-cell-)hole pattern structure of the first (detachable) part
- 29: Frame structure of the first (detachable) part

- 30: Second (detachable) part
- 32: First surface of the second (detachable) part
- 34: Second surface of the second (detachable) part
- 36: Recessed portion of the second (detachable) part
- 38: (Bee-cell-)hole pattern structure of the second (detachable) part
- 39: Frame structure of the second (detachable) part

- 40: Locking element(s)
- 42: First clip element
- 44: Second clip element
- 46: recess in the outer part of the frame structure of the first (detachable) part
- 48: recess in the outer part of the frame structure of the second (detachable) part
- 50: Adjustment zone
- 52: Adjustment zone recess
- 54: Adjustment zone recess
- 56: Form-fit protrusion in the inner part of the frame structure of the first (detachable) part
- 58: Form-fit recess in the inner part of the frame structure of the second (detachable) part

- 100: Surgical lead
- 102: Thin-film lead paddle
- 104: Lead cable
- 106: Interconnection portion
- 108: Electrode area of the thin-film lead paddle
- 110: Protection element (soft material, soft filler)

- S: System

## Claims

1. A protective casing (10) for a thin-film lead paddle (102) of a surgical lead (100), the thin-film lead paddle (102) being connected to a lead cable (104) through an interconnection portion (106) or the thin-film lead paddle (102) being made in one piece with the lead cable (104), the protective casing (10) comprising:
a housing (12) comprising or including a first part (20) and at least a second part (30), the housing (12) being configured and adapted to enclose at least a part of the thin-film lead paddle (102) of the surgical lead (100),
wherein each of the first and second parts (20, 30) includes a first surface (22, 32) and a second surface (24, 34), the second surfaces (24, 34) of the first and second parts (20, 30) being at least partially in contact to one another and/or in contact with the thin-film lead paddle (102) during use.

2. The protective casing (10) according to claim 1,
**characterized in that**
at least one of the first and second parts (20, 30) forming the housing (12) is permeable to fluids and/or gases, especially when the thin-film lead paddle of the surgical lead (100) is placed in the protective casing (10) and being sterilized and/or tested in the protective casing (10).

3. The protective casing (10) according to claim 1 or 2,
**characterized in that**
the first and second parts (20, 30) are detachable, or
the first and second parts (20, 30) are connected to one another through a connection means, preferably wherein said connection means includes a hinge.

4. The protective casing (10) according to claim 1 or 2,
**characterized in that**
the housing (12) is made in one piece.

5. The protective casing (10) according to any one of claims 1 to 3,
**characterized in that**
the protective casing (10) comprises at least one locking element (40) for holding the first and second parts (20, 30) in place with respect to one another to form the housing (10).

6. The protective casing (10) according to claim 5,
**characterized in that**
the at least one locking element (40) is formed integral with at least one of the first and second parts (20, 30) forming the housing (12).

7. The protective casing (10) according to claim 5 or 6,
**characterized in that**
the at least one locking element (40) comprises or consists of at least one clip element (42, 44), especially wherein the clip element (42, 44) is configured to hold the first and second parts (20, 30) in place by clipping.

8. The protective casing (10) according to any one of claims 2 to 7,
**characterized in that**
the first surface (22, 32) and/or the second surface (24, 34) of the first and second parts (20, 30) forming the housing (12) includes a hole pattern structure (28, 38), especially a bee-cell-hole pattern structure (28, 38).

9. The protective casing (10) according to claim 8,
**characterized in that**
the hole pattern structure (30) is formed over almost the entire first surface (22, 32) and/or almost the entire second surface (24, 34) of the first part (20) and/or the second part (30).

10. The protective casing (10) according to any one of the preceding claims,
**characterized in that**
the first part (20) and the second part (30) forming the housing (12) are made of a biocompatible polymer material, preferably a medical grade polymer.

11. The protective casing (10) according to claim 10,
**characterized in that**
the polymer material is Polyamide (PA), especially PA 2200, Polypropylene (PP), Poly ethylene (PE), Poly ethylene terephthalate (PET), Poly-ether-ether-ketone (PEEK), Polyphenylsulfon (PPSU), Polyoxymethylene (POM), Polyethylenimin (PEI).

12. The protective casing (10) according to any one of claims 1 to 3 and 5 to 11,
**characterized in that**
the first and second parts (20, 30) forming the housing (12) comprise a respective recessed portion (26, 36) formed on the second surface (24, 34),
especially wherein the recessed portion (26) of the first part (20) and the recessed portion (36) of the second part (30) define a cavity for housing an interconnection portion (106) when the first and second parts (20, 30) are held in place to form the housing (12).

13. The protective casing (10) according to any one of claims 1 to 3 and 5 to 12,
**characterized in that**
at least one part (20) among the first and second parts (20, 30) comprises a form-fit protrusion (56) and the other part (30) among the first and second parts (20, 30) comprises a form-fit recess (58),
wherein the form-fit protrusion (56) and the form-fit recess (58) are configured to engage with each other for a form-fit when the first and second parts (20, 30) are held in place to form the housing (12).

14. The protective casing (10) according to any one of claims 1 to 3 and 5 to 13,
**characterized in that**
the protective casing (10) comprises an adjustment zone (50) for holding the lead cable (104), wherein the adjustment zone (50) is at least partially formed by the first and second parts (20, 30), especially wherein the first part (20) and/or the second part (30) comprise an adjustment zone recess (52, 54).

15. The protective casing (10) according to any one of the preceding claims,
**characterized in that**
the protective casing (10) further includes an optical inspection window, preferably wherein said optical inspection window is made of a transparent polymer material, more preferably, a biocompatible transparent polymer material.

16. The protective casing (10) according to any one of the preceding claims,
**characterized in that**
a barcode or a radio-frequency identification (RFID) code is provided on the protective casing (10) for traceability.

17. A system (S) comprising:
a surgical lead (100) comprising a thin-film lead paddle (102) and a lead cable (104), especially connected through an interconnecting portion (106) or made in one piece, and
the protective casing (10) according to any one of claims 1 to 16.

18. The system (S) according to claim 17,
**characterized in that**
the system (S) further includes a protection element (110) made of a soft material or a soft filler especially arranged and/or provided at the connection point between the housing (12) and the lead cable (104) of the surgical lead (100).

19. A method for handling a surgical lead (100) comprising a thin-film lead paddle (102), comprising at least the steps of
- covering the thin-film lead paddle (102) with a protective casing (10), especially wherein the protective casing (10) is a protective casing (10) according to one of claims 1 to 16;
- handling the thin-film lead paddle (102) while being in the protective casing (10).

20. A method for sterilizing a surgical lead (100) comprising a thin-film lead paddle (102), comprising at least the steps of
- covering the thin-film lead paddle (102) with a protective casing (10), especially wherein the protective casing (10) is a protective casing (10) according to one of claims 2 to 16
- performing a sterilization operation of the thin-film lead paddle (102) while being in the protective casing (10) by allowing a sterilizing fluid or a sterilizing gas to penetrate inside the casing (10) through the permeable first part (20) and/or second part (30).
